# EUROPEAN PATENT APPLICATION

(11) **EP 4 155 413 A1**
(43) Date of publication of application: **29.03.2023**
(21) Application number: 21818510.6
(22) Date of filing: 02.06.2021
(51) Int. Cl.: C12Q 1/26, C12Q 1/28, C12Q 1/30, C12Q 1/44

(54) **METHOD AND KIT FOR QUANTIFYING LIPOPROTEIN CHOLESTEROL**

(30) Priority: 02.06.2020 JP 2020096248
(71) Applicant: Denka Company Limited, Tokyo 103-8338 (JP)
(72) Inventor: ITOH Yasuki, Tokyo 103-8338 (JP)
(74) Representative: Dietz, Christopher Friedrich
(86) International application number: PCT/JP2021/020923
(87) International publication number: WO 2021/246426

(57) **Abstract**

This invention provides a method for quantification of lipoprotein cholesterol in two steps using an autoanalyzer without pretreatment of an analyte, wherein spontaneous color development of a reagent during storage is suppressed, a kit for quantification used in the method, and a method for preparing such kit. The kit for quantification of lipoprotein cholesterol in a sample obtained from a subject used in the method for quantification of lipoprotein cholesterol in two steps comprises: (1) a first reagent composition comprising cholesterol esterase, cholesterol oxidase, cholesterol esterase, and cholesterol oxidase and leading lipoprotein cholesterol other than the analyte to the outside of the reaction system; and (2) a second reagent composition for quantifying the analyte lipoprotein cholesterol, wherein either the first reagent composition or the second reagent composition comprises at least a coupler, an iron complex, peroxidase, catalase, a hydrogen donor, and a surfactant, provided that the coupler and the hydrogen donor are not allowed to be present in the same reagent composition, and the coupler, the iron complex, and peroxidase are not allowed to be present together in either of the first reagent composition or the second reagent composition.

## Description

### Technical Field

The present invention relates to a method and a reagent for measuring lipoprotein cholesterol.

### Background Art

Cholesterol is an important constituent of cells and is also a clinically important constituent since an excessive level of cholesterol causes transformation of macrophages into foam cells after uptake thereof by macrophages in the subendothelial space and then causes development of a primary lesion of arteriosclerosis. Cholesterol is carried in the form of lipoprotein in the blood, and there are various types of lipoproteins with different functions, such as VLDL, HDL, and LDL. Such various lipoproteins are further divided into subfractions. A lipoprotein subfraction, HDL, is further classified into HDL3 with smaller particle size and higher density and HDL2 with larger particle size and lower density. HDL can be classified into apoE-containing HDL and apoE-deficient HDL depending on different apolipoprotein E (apoE) contents. There is a remnant VLDL with a size reduced from a general size VLDL as a result of degradation by lipoprotein lipase.

Cholesterol in lipoproteins including such subfractions can be assayed using a common autoanalyzer.

Techniques for assaying lipoprotein cholesterol have been reported (see Patent Documents 1 to 5).

### Prior Art Documents

### Patent Documents

Patent Document 1: JP Patent No. 3,164,829
Patent Document 2: JP Patent No. 3,058,602
Patent Document 3: JP Patent No. 5,706,418
Patent Document 4: JP Patent No. 5,706,418
Patent Document 5: JP Patent No. 6,054,051

### Summary of the Invention

### Objects to Be Attained by the Invention

An example of an assay kit for lipoprotein cholesterol including lipoprotein subfractions is, in general, an assay kit comprising a first reagent used in a first step and a second reagent used in a second step. In such assay kit, components necessary for assays are contained separately in the first reagent composition and in the second reagent composition.

However, reagents contained in an assay kit used for quantification of lipoprotein cholesterol are in the form of liquid, such reagents would spontaneously become yellow with the elapse of time, and storage stability of the reagents was an issue of concern.

The present invention provides a method for suppressing spontaneous color development of a reagent during storage in a method for quantification of lipoprotein cholesterol using an autoanalyzer without performing pretreatment of specimens in two steps, and a kit for quantification used in the method for suppressing spontaneous color development. Means for Attaining the Objects

The present inventors have conducted concentrated studies. As a result, they discovered that spontaneous color development of a reagent would be suppressed and reagent stability would be improved by regulating a combination of components, such as an enzyme, a hydrogen donor, a coupler, and an iron complex, to be contained in a first reagent composition used in a first step and in a second reagent composition used in a second step.

Specifically, they discovered that spontaneous color development of a reagent would be suppressed by refraining from allowing a coupler, an iron complex, and peroxidase to be present together in either of two reagent compositions; i.e., a first reagent composition for eliminating or protecting lipoprotein cholesterol other than the analyte and a second reagent composition for quantifying lipoprotein cholesterol, and, in particular, by allowing the coupler and the iron complex to be present separately from each other in such 2 reagent compositions. This has led to the completion of the present invention.

Specifically, the present invention is as described below.
[1] A kit for quantification of lipoprotein cholesterol in a sample obtained from a subject used in a method for quantification of lipoprotein cholesterol in two steps comprising:
   (1) a first reagent composition having cholesterol esterase and cholesterol oxidase and leading lipoprotein cholesterol other than the analyte to the outside of the reaction system; and
   (2) a second reagent composition for quantifying the analyte lipoprotein cholesterol,
   wherein either the first reagent composition or the second reagent composition comprises at least a coupler, an iron complex, peroxidase, a hydrogen donor, and a surfactant, provided that the coupler and the hydrogen donor are not allowed to be present in the same reagent composition, and the coupler, the iron complex, and peroxidase are not allowed to be present together in either of the first reagent composition or the second reagent composition.
[2] A kit for quantification of lipoprotein cholesterol in a sample obtained from a subject used in the method for quantification of lipoprotein cholesterol in two steps comprising:
   (1) a first reagent composition having cholesterol esterase and cholesterol oxidase and leading lipoprotein cholesterol other than the analyte to the outside of the reaction system; and
   (2) a second reagent composition for quantifying lipoprotein cholesterol,
      wherein either the first reagent composition or the second reagent composition comprises at least a coupler, an iron complex, peroxidase, a hydrogen donor, and a surfactant, provided that the coupler and the hydrogen donor are not allowed to be present in the same reagent composition, and the coupler and the iron complex are not allowed to be present together in either of the first reagent composition or the second reagent composition.
[3] The kit for quantifying lipoprotein cholesterol according to [1] or [2], wherein the first reagent composition comprises catalase and a coupler and the second reagent composition comprises a hydrogen donor, an iron complex, and peroxidase.
[4] The kit for quantifying lipoprotein cholesterol according to [1] or [2], wherein the first reagent composition comprises peroxidase and a coupler and the second reagent composition comprises a hydrogen donor and an iron complex.
[5] The kit for quantifying lipoprotein cholesterol according to [1], wherein the first reagent composition comprises catalase, a coupler, and an iron complex and the second reagent composition comprises a hydrogen donor and peroxidase.
[6] The kit for quantifying lipoprotein cholesterol according to [1], wherein the first reagent composition comprises peroxidase and a hydrogen donor and the second reagent composition comprises a coupler and an iron complex.
[7] The kit for quantifying lipoprotein cholesterol according to [1] or [2], wherein the first reagent composition comprises catalase, a hydrogen donor, and an iron complex and the second reagent composition comprises peroxidase and a coupler.
[8] The kit for quantifying lipoprotein cholesterol according to [1] or [2], wherein the first reagent composition comprises peroxidase, a hydrogen donor, and an iron complex and the second reagent composition comprises a coupler.
[9] The kit for quantifying lipoprotein cholesterol according to any of [4], [6], and [8], wherein the first reagent composition further comprises catalase.
[10] The kit for quantifying lipoprotein cholesterol according to any of [1] to [9], wherein the first reagent composition further comprises a surfactant that acts on lipoproteins other than the analyte and the second reagent composition further comprises a surfactant that acts at least on the analyte lipoproteins.
[11] The kit for quantifying lipoprotein cholesterol according to any of [1] to [10], wherein the analyte lipoprotein cholesterol is LDL cholesterol, HDL cholesterol, HDL3 cholesterol, remnant cholesterol, or apoE-containing HDL cholesterol.
[12] The kit for quantifying lipoprotein cholesterol according to [10] or [11], wherein the surfactant contained in the first reagent composition comprises a polyoxyethylene polycyclic phenyl ether derivative or a polyoxyethylene-polyoxypropylene block polymer.
[13] The kit for quantifying lipoprotein cholesterol according to [12], wherein the polyoxyethylene polycyclic phenyl ether derivative includes a polyoxyethylene benzyl phenyl ether derivative and/or a polyoxyethylene styrenated phenyl ether derivative.
[14] A method for quantification of lipoprotein cholesterol in a sample obtained from a subject in two steps comprising:
   (1) a first step of leading lipoprotein cholesterol other than the analyte to the outside of the reaction system in the presence of cholesterol esterase and cholesterol oxidase; and
   (2) a second step of quantifying the analyte lipoprotein cholesterol remaining after the first step,

   wherein, in either the step (1) or (2), at least the coupler, the iron complex, the peroxidase, the hydrogen donor, and the surfactant are used, and the coupler and the hydrogen donor are not used in the same step, and
   in either the step (1) or (2), the coupler, the iron complex, and peroxidase are not used simultaneously.
[15] A method for quantification of lipoprotein cholesterol in a sample obtained from a subject in two steps comprising:
   (1) a first step of leading lipoprotein cholesterol other than the analyte to the outside of the reaction system in the presence of cholesterol esterase and cholesterol oxidase; and
   (2) a second step of quantifying the analyte lipoprotein cholesterol remaining after the first step,
   wherein, in either the step (1) or (2), the coupler and the iron complex are not used simultaneously.
[16] The method for quantification of lipoprotein cholesterol according to [14] or [15], wherein the first step involves the use of the catalase and the coupler and the second step involves the use of the hydrogen donor, the iron complex, and the peroxidase.
[17] The method for quantification of lipoprotein cholesterol according to [14] or [15], wherein the first step involves the use of the peroxidase and the coupler and the second step involves the use of the hydrogen donor and the iron complex.
[18] The method for quantification of lipoprotein cholesterol according to [14], wherein the first step involves the use of the catalase, the coupler, and the iron complex and the second step involves the use of the hydrogen donor and the peroxidase.
[19] The method for quantification of lipoprotein cholesterol according to [14], wherein the first step involves the use of the peroxidase and the hydrogen donor and the second step involves the use of the coupler and the iron complex.
[20] The method for quantification of lipoprotein cholesterol according to [14] or [15], wherein the first step involves the use of the catalase, the hydrogen donor, and the iron complex and the second step involves the use of the peroxidase and the coupler.
[21] The method for quantification of lipoprotein cholesterol according to [14] or [15], wherein the first step involves the use of the peroxidase, the hydrogen donor, and the iron complex and the second step involves the use of the coupler.
[22] The method for quantification of lipoprotein cholesterol according to any of [17], [19], and [21], wherein the first step further involves the use of the catalase.
[23] The method for quantification of lipoprotein cholesterol according to any of [14] to [22], wherein the first step further involves the use of a surfactant that acts on lipoproteins other than the analyte and the second step further involves the use of a surfactant that acts at least on the analyte lipoproteins.
[24] The method for quantification of lipoprotein cholesterol according to any of [14] to [23], wherein the analyte lipoprotein cholesterol is LDL cholesterol, HDL cholesterol, HDL3 cholesterol, remnant cholesterol, or apoE-containing HDL cholesterol.

This description includes part or all of the content as disclosed in the description and/or drawings of Japanese Patent Application No. 2020-096248, which is a priority document of the present application.

### Effects of the Invention

The present invention provides a method for stable quantification of lipoprotein cholesterol by suppressing spontaneous color development of 2 reagent compositions during storage to improve reagent stability when subjecting the analyte lipoprotein cholesterol to fractional measurement separately from lipoprotein cholesterol other than the analyte, a kit for quantification used in the method of quantification, and a method for producing such kit used to implement the method of quantification.

### Brief Description of the Drawings

Fig. 1 shows the absorbance spectra immediately after preparation and after storage of the second reagent composition in Comparative Example 1.
Fig. 2 shows the absorbance spectra immediately after preparation and after storage of the first reagent composition in Comparative Example 2.
Fig. 3a shows the absorbance spectra immediately after preparation and after storage of the first reagent composition in Example 1.
Fig. 3b shows the absorbance spectra immediately after preparation and after storage of the second reagent composition in Example 1.
Fig. 4a shows the absorbance spectra immediately after preparation and after storage of the first reagent composition in Example 2.
Fig. 4b shows the absorbance spectra immediately after preparation and after storage of the second reagent composition in Example 2.
Fig. 5a shows the absorbance spectra immediately after preparation and after storage of the first reagent composition in Example 3.
Fig. 5b shows the absorbance spectra immediately after preparation and after storage of the second reagent composition in Example 3.
Fig. 6a shows the absorbance spectra immediately after preparation and after storage of the first reagent composition in Example 4.
Fig. 6b shows the absorbance spectra immediately after preparation and after storage of the second reagent composition in Example 4.
Fig. 7a shows the absorbance spectra immediately after preparation and after storage of the first reagent composition in Example 5.
Fig. 7b shows the absorbance spectra immediately after preparation and after storage of the second reagent composition in Example 5.
Fig. 8a shows the absorbance spectra immediately after preparation and after storage of the first reagent composition in Example 6.
Fig. 8b shows the absorbance spectra immediately after preparation and after storage of the second reagent composition in Example 6.
Fig. 9a shows the absorbance spectra immediately after preparation and after storage of the first reagent composition in Example 7.
Fig. 9b shows the absorbance spectra immediately after preparation and after storage of the second reagent composition in Example 7.
Fig. 10a shows the absorbance spectra immediately after preparation and after storage of the first reagent composition in Example 8.
Fig. 10b shows the absorbance spectra immediately after preparation and after storage of the second reagent composition in Example 8.
Fig. 11a shows the absorbance spectra immediately after preparation and after storage of the first reagent composition in Example 9.
Fig. 11b shows the absorbance spectra immediately after preparation and after storage of the second reagent composition in Example 9.

### Embodiments of the Invention

Hereafter, the present invention is described in detail.

Lipoproteins can be roughly classified into CM, VLDL, LDL, and HDL. Each lipoprotein is further classified into subfractions. These fractions and subfractions may be distinguished from each other based on particle size or density. The particle size (or particle diameter) of VLDL ranges from 30 nm to 80 nm (30 nm to 75 nm), that of LDL ranges from 22 nm to 28 nm (19 nm to 30 nm), that of HDL ranges from 7 nm to 12 nm, that of HDL3 as an HDL subfraction ranges from 7 nm to 8.5 nm, and that of HDL2 ranges from 8.5 nm to 10 nm, although such figures may vary depending on researchers. The density of VLDL is 1.006 or less, that of LDL ranges from 1.019 to 1.063, that of HDL ranges from 1.063 to 1.21, that of HDL2 ranges from 1.063 to 1.125, and that of HDL3 ranges from 1.125 to 1.210. The diameters of particles can be measured by gradient gel electrophoresis (GGE) (JAMA, 260, pp. 1917-21, 1988) or NMR (HANDBOOK OF LIPOPROTEIN TESTING 2nd Edition, Edited by Nader Rifai et al., AACC PRESS: The Fats of Life, Summer 2002, LVDD 15 YEAR ANNIVERSARY ISSUE, Volume AVI No. 3, pp. 15-16). Density can be determined based on analyses by ultracentrifugation (Atherosclerosis, 106, pp. 241-253, 1994: Atherosclerosis, 83, p. 59, 1990). HDL cholesterol (HDL-C) and LDL cholesterol (LDL-C) can be assayed using an autoanalyzer.

HDL can be classified into the ApoE-containing HDL subfraction and the ApoE-deficient HDL subfraction depending on different ApoE contents. In general, the term "ApoE-containing HDL" refers to HDL that comprises ApoE incorporated therein and the term "ApoE-deficient HDL" refers to HDL that does not comprises ApoE incorporated therein. HDL in which ApoE is present or the ApoE content is high may be referred to as "ApoE-rich HDL," and such HDL is also within the scope of "ApoE-containing HDL." Since ApoE fractions are continuously distributed within HDL, ApoE-containing HDL cannot be clearly distinguished from ApoE-deficient HDL on the basis of the ApoE content in lipoproteins. For example, it is possible to quantify apoE-containing HDL cholesterol (apoE-containing HDL-C) by subtracting the results obtained by the phosphotungstic acid-dextran sulfate-magnesium (PT-DS-Mg) precipitation method (BIOCHEMICAL MEDICINE AND METABOLIC BIOLOGY 46, 329-343, 1991) that can selectively assay ApoE-deficient HDL-C without assaying ApoE-containing HDL-C from the results obtained by the 13% PEG method (J. Lipid Research 3, 8, 1204-16: 1997) that can assay total HDL-C including ApoE-containing HDL-C. Alternatively, apoE-containing HDL-C can be assayed using an autoanalyzer based on a difference in reactivity influenced by the surfactant concentration (Ann. Clin. Biochem., 56, 123-132, 2019).

Remnant (remnant-like lipoprotein, RLP) is a special lipoprotein generated when the lipid metabolism is disturbed because of metabolic syndromes and is a metabolic product of CM or VLDL degraded by the action of lipoprotein lipase. While the remnant is not defined based on a particular size or density, it can be quantified with the use of, for example, a gel on which an antibody reacting with a constitutive apoprotein in lipoproteins immobilized thereon, a phospholipid-degrading enzyme, or a surfactant. Alternatively, RLP-cholesterol (RLP-C) can be assayed using an autoanalyzer based on a difference in reactivity influenced by the molecular weight of cholesterol esterase (J. Applied Laboratory Medicine 3, 26-36, 2018).

In the present invention, HDL cholesterol (HDL-C), LDL-cholesterol (LDL-C), HDL3- cholesterol (HDL3-C), remnant cholesterol (RLP-C), and apoE-containing HDL-C are analytes.

The kit for quantification of various lipoproteins according to the present invention is used in the method for quantification of lipoprotein cholesterol in two steps. The first reagent composition of the quantification kit of the present invention is used in the first step and the second reagent composition thereof is used in the second step.

The first reagent composition of the kit for quantifying lipoprotein cholesterol according to the present invention comprises cholesterol esterase and cholesterol oxidase and leads lipoprotein cholesterol other than the analyte to the outside of the reaction system in the presence of cholesterol esterase and cholesterol oxidase.

Examples of ingredients of the first reagent composition that can be used include, but are not limited to, albumin, a buffer, a surfactant, catalase, peroxidase, a surfactant comprising a polyoxyethylene derivative, a hydrogen donor, and a coupler.

The second reagent composition in the kit for quantification of lipoprotein cholesterol according to the present invention comprises ingredients for quantification of the analyte lipoprotein cholesterol. While ingredients of the second reagent composition vary depending on the first reagent composition, a known substance can be used without particular limitation, provided that lipoprotein cholesterol can be quantified.

Examples of ingredients of the second reagent composition that can be used include, but are not limited to, peroxidase, a polyoxyethylene derivative, a hydrogen donor, and a coupler.

The method of the present invention comprises 2 steps. In the first step, a surfactant that can act on lipoproteins other than the analyte is allowed to react with a sample obtained from a subject in the presence of cholesterol esterase and cholesterol oxidase, and cholesterol generated upon liberation from lipoproteins is allowed to react with cholesterol oxidase and led to the outside of the reaction system. In this case, the analyte lipoprotein cholesterol can be prevented from being led to the outside of the reaction system in the presence of a surfactant that protects the analyte. In the subsequent second step, lipoprotein cholesterol remaining unreacted after the first step is quantified.

Either the first reagent composition or the second reagent composition comprises at least a coupler, an iron complex, peroxidase, catalase, a hydrogen donor, and a surfactant, and the coupler and the hydrogen donor would not be present in the same reagent composition.

The method of the present invention is generally carried out within an autoanalyzer.

In the method of the present invention, a hydrogen donor is subjected to coupling with a coupler in the presence of peroxidase when quantifying the lipoprotein cholesterol level, and the absorbance at a particular wavelength is measured based on the generated dye. In such a case, an iron complex is used as a reaction promoter. When all of the coupler, peroxidase, and the iron complex are present in the same reagent composition, however, the reagent would undergo spontaneous color development with the elapse of time, and quantification of lipoprotein cholesterol would be adversely affected. In the present invention, accordingly, either of the coupler, peroxidase, and the iron complex was allowed to be present in another reagent composition, so that the reagent could be prevented from undergoing spontaneous color development and stabilized. When "peroxidase is present," an enzyme having peroxidase activity is present, and a reaction catalyzed by peroxidase can occur. The expression "peroxidase is present" can be understood as "peroxidase activity is present." The same applies to cholesterol esterase, cholesterol oxidase, and catalase. When a reagent composition comprises peroxidase, cholesterol esterase, cholesterol oxidase, and catalase, a catalytic reaction necessary for assays takes place when such enzymes exert activity. Accordingly, the presence of such enzymes can be demonstrated by assaying such enzyme activity.

In the method of the present invention, the coupler, peroxidase activity, and the iron complex are allowed to be present in the first reagent composition used in the first step and the second reagent composition used in the second step separately from each other as described below.
(1) The coupler is allowed to be present in the first reagent composition used in the first step, and peroxidase activity and the iron complex are allowed to be present in the second reagent composition used in the second step.
(2) The coupler and peroxidase activity are allowed to be present in the first reagent composition and the iron complex is allowed to be present in the second reagent composition.
(3) Peroxidase activity is allowed to be present in the first reagent composition and the coupler and the iron complex are allowed to be present in the second reagent composition.
(4) The coupler and the iron complex are allowed to be present in the first reagent composition and peroxidase activity is allowed to be present in the second reagent composition.
(5) Peroxidase activity and the iron complex are allowed to be present in the first reagent composition and the coupler is allowed to be present in the second reagent composition.
(6) The iron complex is allowed to be present in the first reagent composition and peroxidase activity and the coupler are allowed to be present in the second reagent composition.

In addition, the coupler and the hydrogen donor are preferably allowed to be present separately from each other in different reagent compositions.

Hereafter, each step is described in detail.

The kit of the present invention comprises a first reagent composition used in a step of leading lipoprotein cholesterol other than the analyte to the outside of the reaction system and a second reagent composition used in a step of measuring the analyte lipoprotein cholesterol.

In the step of leading lipoprotein cholesterol other than the analyte to the outside of the reaction system, conventional techniques, such as a technique of eliminating lipoprotein cholesterol other than the analyte and leading such cholesterol to the outside of the reaction system and a technique of allowing lipoprotein cholesterol other than the analyte to aggregate or inhibiting such cholesterol from undergoing a reaction in the subsequent step, can be employed.

The first step of eliminating lipoprotein cholesterol other than the analyte and leading such cholesterol to the outside of the reaction system is carried out in the manner described below:
(1) a method of generating hydrogen peroxide from lipoprotein cholesterol with the aid of cholesterol esterase and cholesterol oxidase and degrading hydrogen peroxide into water and oxygen in the presence of catalase;
(2) a method of forming colorless quinine in the presence of hydrogen peroxide generated with the aid of cholesterol esterase and cholesterol oxidase and a coupler or a hydrogen donor; or
(3) a method of degrading hydrogen peroxide in the presence of catalase and, at the same time, forming colorless quinine in the presence of a coupler or a hydrogen donor.

In the method (1) of degrading hydrogen peroxide in the presence of catalase, it is necessary that catalase is present in the first reagent composition used in the first step. In the method (2) of forming colorless quinine, in addition, it is necessary that at least one of a coupler and a hydrogen donor is present in the first reagent composition and that peroxidase is further present in the first reagent composition. In the method (3) of degrading hydrogen peroxide with the aid of catalase and, at the same time, forming colorless quinine in the presence of a coupler or a hydrogen donor, it is necessary that catalase, peroxidase, and a coupler or a hydrogen donor are present in the first reagent composition.

Examples of couplers that can be used in the coupling reaction in the present invention include, but are not limited to, 4-aminoantipyrine, aminoantipyrine derivative, vanillin diamine sulfonic acid, methylbenzothiazolinone hydrazone, and sulfonated methylbenzothiazolinone hydrazone.

A hydrogen donor that is preferably used in the present invention is an aniline derivative. Examples of such aniline derivative include N-ethyl-N-(2-hydroxy-3-sulfopropyl)-3 -methylaniline (TOO S), N-ethyl-N-(2-hydroxy-3 -sulfopropyl)-3,5-dimethylaniline (MAOS), N-ethyl-N-(3-sulfopropyl)-3-methylaniline (TOPS), N-(2-hydroxy-3-sulfopropyl)-3,5-dimethoxyaniline (HDAOS), N-(3-sulfopropyl)aniline (HALPS), N-(3-sulfopropyl)-3-methoxy-5-aniline (HMMPS), N-ethyl-N-(2-hydroxy-3-sulfopropyl)-4-fluoro-3,5-dimethoxyaniline (FDAOS), and N-ethyl-N-(3-methylphenyl)-N'-succinylethylenediamine (EMSE). The final concentration of such hydrogen donor to be used in a reaction solution preferably ranges from 0.1 mmol/l to 8 mmol/l.

Examples of iron complexes used in the present invention include potassium ferrocyanide, sodium ferrocyanide, a porphyrin-iron complex, and an EDTA-iron complex. A preferable concentration of the iron complex is 0.001 to 0.05 mmol/l.

In the method of the present invention, the first step of eliminating lipoprotein cholesterol other than the analyte and leading such cholesterol to the outside of the reaction system involves the use of a surfactant comprising a polyoxyethylene derivative. When assaying HDL3-C and RLP-C in the present invention, the first reagent composition used in the first step may comprise sphingomyelinase. When "sphingomyelinase is present," an enzyme having sphingomyelinase activity is present, and a reaction of degrading sphingomyelin can occur. The expression "sphingomyelinase is present" can be understood as "sphingomyelinase activity is present." When phospholipase, such as phospholipase C or phospholipase D, has sphingomyelinase activity, such enzymes are within the scope of "sphingomyelinase." The sphingomyelinase concentration in the reagent is preferably 0.1 to 100 U/ml, and more preferably 0.2 to 20 U/ml. The concentration in the reaction solution during the reaction is preferably 0.05 to 100 U/ml, and more preferably 0.1 to 20 U/ml. Specific examples of preferable sphingomyelinase include, but are not limited to, SPC (Asahi Kasei Corporation), Sphingomyelinase from *Bacillus cereus,* and Sphingomyelinase from *Staphylococcus aureus* (SIGMA).

In the step of eliminating lipoprotein cholesterol other than the analyte, lipoprotein cholesterol other than the analyte is eliminated in the presence of a surfactant that acts on lipoproteins other than the analyte but does not react with the analyte lipoprotein. An example of a surfactant that acts on and reacts with lipoproteins other than the analyte is a polyoxyethylene derivative. Examples of polyoxyethylene derivatives include polyoxyethylene alkyl ether, a polyoxyethylene polycyclic phenyl ether derivative, and a polyoxyethylene-polyoxypropylene block polymer. Preferable examples of polyoxyethylene polycyclic phenyl ether derivatives include a polyoxyethylene benzylphenyl derivative, a sulfate thereof, a polyoxyethylene styrenated phenyl ether derivative, a sulfate thereof, and specific phenol ethoxylate. Specific examples of polyoxyethylene polycyclic phenyl ether derivatives include: EMULGEN A-60, EMULGEN A-500, EMULGEN B-66, and EMULGEN A-90 (Kao Corporation); Newcol 703, Newcol 704, Newcol 706, Newcol 707, Newcol 708, Newcol 709, Newcol 710, Newcol 711, Newcol 712, Newcol 714, Newcol 719, Newcol 723, Newcol 729, Newcol 733, Newcol 740, Newcol 747, Newcol 780, Newcol 610, Newcol 2604, Newcol 2607, Newcol 2609, and Newcol 2614 (NIPPON NYUKAZAI CO., LTD.); Noigen EA-87, Noigen EA-137, Noigen EA-157, Noigen EA-167, Noigen EA-177, Noigen EA-197D, and Noigen EA-207D (Dai-ichi Kogyo Seiyaku); and Blaunon DSP-9, Blaunon DSP-12.5, Blaunon TSP-7.5, Blaunon TSP-16, and Blaunon TSP-50 (Aoki Oil Industrial Co., Ltd.). Specific examples of polyoxyethylene polycyclic phenyl ether and sulfate thereof include: HITENOL NF-08, HITENOL NF-13, HITENOL NF-17, and HITENOL NF0825 (Dai-ichi Kogyo Seiyaku); and Newcol 707-SF, Newcol 707-SFC, Newcol 707-SN, Newcol 714-SF, Newcol 714-SN, Newcol 723-SF, Newcol 740-SF, Newcol 780-SF, Newcol 2607-SF, and Newcol 2614-SF (NIPPON NYUKAZAI CO., LTD.). The concentration of a surfactant in a reagent is preferably 0.3% to 5% (w/v), and more preferably 0.5% to 3% (w/v). The concentration in the reaction solution during the reaction is preferably 0.15% to 5%, and more preferably 0.25% to 3% (w/v). A surfactant in a reagent can be identified by a method of analysis comprising performing, for example, IR, NMR, and LC-MS in adequate combination. Examples of methods of identifying the ionic property of a surfactant (i.e., nonionic, anionic, or cationic property) include an extraction method and a solid-phase extraction method using an organic solvent under acidic or alkaline conditions. A surfactant structure can be analyzed by LC-MSMS or NMR.

In the above step of eliminating lipoprotein cholesterol other than the analyte, cholesterol esterase acts on lipoproteins other than the analyte in the presence of a surfactant that acts on lipoproteins other than the analyte, and the resulting cholesterol is allowed to react and eliminated in the presence of an enzyme that reacts with cholesterol, such as cholesterol oxidase. Thus, lipoprotein cholesterol is led to the outside of the reaction system. When "a surfactant acts on (reacts with)," a surfactant degrades lipoproteins and liberates lipoprotein cholesterol. For example, "a surfactant that acts on (reacts with) lipoproteins other than the analyte" is not required to be completely unreactive with lipoproteins, but it may be mainly reactive with lipoproteins other than the analyte. The term "elimination" means to degrade a substance in a sample obtained from a subject, so as to prevent the degraded product from being detected in the subsequent step. When "eliminating lipoprotein cholesterol other than the analyte," specifically, lipoproteins other than the analyte in a sample obtained from a subject are degraded, and the degraded product; that is, lipoprotein cholesterol, is prevented from being detected in the subsequent step.

In the present invention, the phrase "led (or ..leading...) to the outside of the reaction system" refers to a system in which cholesterol contained in lipoproteins other than the analyte is eliminated, aggregated, or inhibited to avoid its reaction in the subsequent steps, so that such cholesterol would not affect quantification of the analyte lipoprotein cholesterol.

A person skilled in the art can select an adequate surfactant to lead lipoprotein cholesterol other than the analyte lipoprotein cholesterol to the outside of the reaction system. For example, use of surfactants in accordance with the analyte lipoprotein cholesterol is preferable.
HDL-C: Polyoxyethylene-polyoxypropylene block polymer
LDL-C: Polyoxyethylene polycyclic phenyl ether
HDL3-C: Polyoxyethylene-polyoxypropylene block polymer
Remnant -C: Polyoxyethylene polycyclic phenyl ether
apoE-containing HDL-C: Polyoxyethylene polycyclic phenyl ether

Cholesterol esterase and cholesterol oxidase are caused to act in the presence of the above surfactant to generate hydrogen peroxide from cholesterol and the generated hydrogen peroxide is then eliminated. The concentration of cholesterol esterase in a reaction solution preferably ranges from approximately 10 U/l to 1000 U/l. Also, cholesterol oxidase derived from a bacterium or yeast can be used. The concentration of cholesterol oxidase in a reaction solution preferably ranges from approximately 100 U/l to 3500 U/l. The concentration of catalase in a reaction solution preferably ranges from approximately 40 U/l to 2500 U/l. When converting hydrogen peroxide into colorless quinine, the concentration of peroxidase in a reaction solution preferably ranges from 400 U/l to 2000 U/l.

In the step of eliminating lipoprotein cholesterol other than the analyte, cholesterol in the lipoproteins that had acted on and reacted with the surfactant and cholesterol esterase can be allowed to react with a cholesterol-reactive enzyme, such as cholesterol oxidase, and led to the outside of the reaction system. Cholesterol in lipoproteins other than the analyte can be led to the outside of the reaction system through the reaction as described above. In the subsequent step, accordingly, the analytes would selectively remain as lipoproteins in the reaction solution. In the present invention, lipoproteins other than the analyte are eliminated and led to the outside of the reaction system, so as to prevent lipoprotein cholesterol other than the analyte from being detected in the subsequent step. This process may be referred to as "differentiation of the analyte lipoproteins from lipoproteins other than the analyte."

In the step of eliminating lipoprotein cholesterol other than the analyte, it is not necessary that lipoprotein cholesterol other than the analyte is completely eliminated. In such a case, a surfactant that enables selective measurement of lipoprotein cholesterol may be used in a step of quantifying the analyte lipoprotein cholesterol.

In the step of eliminating lipoprotein cholesterol other than the analyte and leading such cholesterol to the outside of the reaction system, the concentration of cholesterol esterase in the reaction solution is preferably 10 U/l to 10000 U/l, more preferably 300 U/l to 2500 U/l, and particularly preferably 600 U/l to 2000 U/l. Cholesterol esterase used in the present invention is not particularly limited, provided that such enzyme can hydrolyze cholesterol ester. Animal- or microbe-derived cholesterol esterase can be used.

A reaction solution used in the step of eliminating lipoprotein cholesterol other than the analyte can be supplemented with a lipoprotein-degrading enzyme, according to need, so as to regulate the activity on various lipoproteins. An example of a lipoprotein-degrading enzyme that can be used is lipoprotein lipase. Lipoprotein lipase is not particularly limited, provided that such enzyme can degrade a lipoprotein. Animal- or microbe-derived lipoprotein lipase can be used. The concentration of lipoprotein lipase in the reaction solution is preferably 10 U/l to 10000 U/l, more preferably 10 U/l to 5000 U/l, and particularly preferably 10 U/l to 1000 U/l.

In the present invention, lipoprotein cholesterol that has remained unreacted in the step of leading lipoprotein cholesterol other than the analyte to the outside of the reaction system is quantified in the subsequent second step. A conventional method for quantification can be used in the step of quantification. Examples of methods include a method for quantification of the content of the specific aggregate formed with the addition of a coagulant by turbidimetric determination, a method involving the use of an antigen-antibody reaction with a specific antibody, and a method for quantification of a degraded product with the use of an enzyme. Among such methods, the method for quantification of a degraded product with the use of an enzyme is preferable.

In such method, an enzyme for cholesterol assays, such as cholesterol esterase or cholesterol oxidase, is added to liberate and degrade lipoprotein cholesterol, and the reaction products is then quantified. When lipoprotein cholesterol other than the analyte has been led to the outside of the reaction system in the first step of quantification, a surfactant that acts on at least the analyte lipoprotein may be used to quantify the analyte lipoprotein cholesterol. A surfactant that acts on at least the analyte lipoprotein may be a surfactant that selectively acts on the analyte lipoprotein, a surfactant that also acts on lipoproteins other than the analyte lipoproteins, or a surfactant that acts on all lipoproteins.

When lipoprotein cholesterol other than the analyte remains uneliminated in the first step, in the second step, it is necessary to use a surfactant that selectively reacts with the analyte lipoproteins among the remaining lipoproteins.

An example of a surfactant that acts on all lipoproteins is a polyoxyethylene derivative. Any commercially available surfactant that is used in reagents or the like for total cholesterol measurement can be used. Specific examples include polyoxyethylene alkyl phenyl ether (EMULGEN 909 (Kao Corporation) and TritonX-100) and polyoxyethylene alkyl ether (EMULGEN 707 (Kao Corporation) and EMULGEN 709 (Kao Corporation)).

The concentration of a surfactant in a reaction solution to be used in the step of quantification of lipoproteins preferably ranges from approximately 0.01% (w/v) to 10% (w/v) and more preferably ranges from approximately 0.1% (w/v) to 5% (w/v).

Cholesterol esterase activity, cholesterol oxidase activity, sphingomyelinase activity, peroxidase activity, and catalase activity used in the present invention can be measured by the methods described below. The description below is merely an example, and enzyme activity can be measured by any conventional method.

Cholesterol oxidase activity is measured using a 6 mM cholesterol solution (dissolved in isopropanol) as a substrate solution. A diluent (0.1 M phosphate buffer, TritonX-100, pH 7.0) is added to adjust the concentration of the analyte to 2 to 4 U/ml, 3 ml of the diluted solution is heated at 37°C for 5 minutes, and 0.05 ml of a substrate solution is then added. Thereafter, the mixture is subjected to the reaction at 37°C, and changes in the absorbance at 240 nm are measured. In the present invention, changes in the absorbance are measured 2 to 7 minutes after the reaction at 37°C to determine cholesterol oxidase activity. If enzyme activity per unit amount converted from the changes in the absorbance measured in the manner described above is 3 U/l or higher, it can be considered that cholesterol oxidase activity is present in the analyte. In other words, the analyte contains "cholesterol oxidase."

Cholesterol esterase activity is measured using a substrate solution (a solution of 0.04% linolenic acid cholesterol, 1% TritonX-100, and 0.6% sodium cholate), a 300 U/ml cholesterol oxidase solution, an enzyme diluent (20 mM phosphate buffer, 0.5 mM EDTA · 2Na, 2 mM MgCl₂, 0.2% BSA, pH 7.5), and a reaction solution (0.06% 4-aminoantipyrine, 0.4% phenol, 7.5 KU/l peroxidase). After 1.75 ml of the reaction solution is mixed with 1.0 ml of the substrate solution, the mixture is heated at 37°C for 5 minutes, and 0.1 ml of a cholesterol oxidase solution is added. After the mixture is heated at 37°C for 2 minutes, 0.1 ml of the analyte diluted with a diluent is added, the mixture is subjected to the reaction at 37°C, and changes in the absorbance at 500 nm is then measured. In the present invention, changes in the absorbance are measured 0 to 3.5 minutes after the reaction at 37°C to determine cholesterol esterase activity. If enzyme activity per unit amount converted from the changes in the absorbance measured in the manner described above is 8 U/l or higher, it can be considered that cholesterol esterase activity is present in the analyte. In other words, the analyte contains "cholesterol esterase."

Peroxidase activity is measured using a reaction solution 1 (1.5 mM HDAOS, 0.05% TritonX-100, 50 mM phosphate buffer, pH 7.0), a reaction solution 2 (5 mM 4-aminoantipyrine, 0.05% TritonX-100, 1% hydrogen peroxide, 50 mM phosphate buffer, pH 7.0), and a diluent (50 mM phosphate buffer, pH 7.0). The reaction solution 1 (0.3 ml) is mixed with 0.08 ml of the analyte diluted with a diluent, and the mixture is heated at 37°C for 5 minutes. Thereafter, 0.1 ml of the reaction solution 2 is added, the reaction is allowed to proceed at 37°C, and changes in the absorbance are measured at a dominant wavelength of 600 nm and a subwavelength of 700 nm. In the present invention, changes in the absorbance are measured 2 to 5 minutes after the reaction at 37°C to determine peroxidase activity. If enzyme activity per unit amount converted from the changes in absorbance measured in the manner described above is 10 U/l or higher, it can be considered that peroxidase activity is present in the analyte. In other words, the analyte contains "peroxidase."

Catalase activity is measured using a substrate solution (0.06% hydrogen peroxide, 50 mM phosphate buffer, pH 7.0). After 2.9 ml of the substrate solution is pre-heated at 25°C, the solution is mixed with 0.1 ml of the analyte, and changes in absorbance at 240 nm are measured. In the present invention, changes in absorbance are measured 0 to 3 minutes after the reaction at 25°C to determine catalase activity. If enzyme activity per unit amount converted from the changes in absorbance measured in the manner described above is 100 U/l or higher, it can be considered that catalase activity is present in the analyte. In other words, the analyte contains "catalase."

Sphingomyelinase activity is measured using a reaction solution (a mixture of 0.008% sphingomyelin, a 0.05% TritonX-100 solution, 10 U/ml alkaline phosphatase, 10 U/ml cholesterol oxidase, 2 U/ml peroxidase, 0.02% 4-aminoantipyrine, and 0.02% TODB), a reaction terminator (a 1% sodium dodecyl sulfate solution), and a diluent (10 mM Tris buffer, 0.1% TritonX-100, pH 8.0). The reaction solution (0.08 ml) is mixed with 0.003 ml of the analyte diluted with a diluent, the mixture is heated at 37°C for 5 minutes, and 0.16 ml of the reaction terminator is then added. After the reaction is terminated, changes in absorbance are measured at a dominant wavelength of 546 nm and a subwavelength of 700 nm to determine sphingomyelinase activity. If enzyme activity per unit amount converted from the changes in absorbance measured in the manner described above is 2 U/l or higher, it can be considered that sphingomyelinase activity is present in the analyte. In other words, the analyte contains "sphingomyelinase."

In the step of eliminating lipoprotein cholesterol other than the analyte and leading such cholesterol to the outside of the reaction system according to the present invention, a monovalent cation and/or a divalent cation or a salt thereof can be used as an ionic strength adjuster. With the addition of such ionic strength adjuster, the analyte lipoproteins can be easily differentiated from other lipoproteins. Specifically, sodium chloride, potassium chloride, magnesium chloride, manganese chloride, calcium chloride, lithium chloride, ammonium chloride, sodium sulfate, magnesium sulfate, potassium sulfate, lithium sulfate, ammonium sulfate, magnesium acetate, and the like can be used. The concentration of such ionic strength adjuster at the time of the reaction preferably ranges from 0 mM to 100 mM.

The enzyme in the reagent can also be identified in the manner described below. Specifically, a sample containing a target enzyme is degraded with trypsin, and the resulting fragment peptide is detected using a hybrid mass spectrometer. The peptide mass determined using a mass spectrometer and the spectra of fragment ions obtained in collision with argon gas within a mass spectrometer (MS/MS data) are applied to database search (e.g., Mascot search). Thus, a protein can be identified. When a sequence of a fragment peptide derived from an amino acid sequence in the sample is consistent with the amino acid sequence registered with the database as an unique sequence, the sample can be considered to contain a target enzyme.

The enzyme in the reagent can also be identified in the manner described below. From among fragment peptides obtained by degrading a target enzyme with trypsin, specifically, a peptide that is specific to a target enzyme and exhibits an intense signal detected by mass spectrometry is selected as a target peptide to be quantified. Concerning the target peptide to be quantified, a non-labeled peptide and a peptide labeled with a stable isotope as the internal standard are prepared via chemical synthesis. A sample containing a target enzyme is completely digested with trypsin, a known amount of a stable isotope-labeled peptide is added, and the sample is analyzed using a triple quadrupole mass spectrometer (LC-MS/MS) connected to HPLC in the MRM mode (i.e., the multiple reaction monitoring mode). A mixture containing a non-labeled target peptide and a known amount of a stable isotope-labeled peptide is analyzed in the same manner, a calibration curve indicating the concentration ratio and the peak area ratio relative to the internal standard is prepared, and the absolute amount of the target peptide in the sample is determined. Thus, the target enzyme can be quantified.

The reaction in each step of the present invention is preferably carried out at a temperature between 2°C and 45°C and more preferably between 25°C and 40°C.

The reaction in each step is preferably carried out for 1 to 10 minutes and more preferably for 3 to 7 minutes.

Serum and blood plasma samples can be used as samples obtained from a subject in the present invention, although samples are not limited thereto.

Examples of an autoanalyzer to be used in the present invention include TBA-120FR 200FR (TOSHIBA Corporation), JCA-BM 1250 1650 2250 (JEOL Ltd.), HITACHI 7180 7170 (Hitachi, Ltd.), AU 2700 - 5800 - 680 (OLYMPUS), and cobas c501 · 701 (Roche).

When there are 2 steps; i.e., a step of eliminating lipoprotein cholesterol other than the analyte and leading such cholesterol to the outside of the reaction system and a step of measuring the analyte lipoproteins, a reagent composition used in the step of eliminating lipoprotein cholesterol other than the analyte and leading such cholesterol to the outside of the reaction system contains a surfactant that reacts with lipoproteins other than the analyte. A reagent composition used for eliminating lipoprotein cholesterol other than the analyte lipoproteins may further be supplemented with an enzyme that degrades cholesterol, such as cholesterol esterase or cholesterol oxidase, either of a hydrogen donor or a coupler, catalase for eliminating hydrogen peroxide, and the like. A reagent composition used in the step of measuring the analyte lipoproteins may be supplemented with a surfactant that selectively reacts with the analyte lipoproteins or a surfactant that reacts with all lipoproteins and either of a hydrogen donor or a coupler, which is not used in the step of eliminating lipoprotein cholesterol other than the analyte lipoproteins and leading such cholesterol to the outside of the reaction system. In such a case, a monovalent cation, a divalent cation, a salt of the monovalent or divalent cation, or a polyanion may also be added to the first reagent composition or the second reagent composition, according to need. Also, the first reagent composition or the second reagent composition may be supplemented with serum albumin. The pH of each reagent composition may be around neutral and ranges, for example, from 6 to 8 and preferably ranges from 6.5 to 7.5. The pH may be adjusted with the addition of a buffer.

When the method of the present invention comprises performing the step of eliminating lipoprotein cholesterol other than the analyte and leading such cholesterol to the outside of the reaction system and the step of measuring the analyte lipoproteins in that order, a reagent composition used for eliminating lipoprotein cholesterol other than the analyte may be first added to a sample obtained from a subject for a reaction, a reagent composition used for measuring the analyte lipoproteins may be added for a further reaction, and the absorbance may then be measured.

The amount of a sample obtained from a subject and the amount of each reagent composition are not particularly limited, and such amounts can be adequately determined in view of the concentration or the like of a reagent in each reagent composition, provided that the sample and the reagent composition can be applied to an autoanalyzer. For example, 1 µl to 10 µl of a sample obtained from a subject, 50 to 300 µl of the first reagent, and 25 to 200 µl of the second reagent may be used.

### Examples

Hereafter, the present invention is described in greater detail with reference to the examples, although the present invention is not limited to the examples described below.

Ingredients used in Examples and Comparative Examples are as described below.

In Examples and Comparative Examples below, absorbance spectra were measured using HITACHI U-3900.

Types and concentrations of substances other than a hydrogen donor, a coupler, catalase, potassium ferrocyanide, and peroxidase that are contained in the first reagent composition or the second reagent composition for various analytes are as described below.

### Analyte: HDL-C

First reagent composition
   BES buffer (pH 7.0): 100 mM
   Cholesterol esterase: 800 U/l
   Cholesterol oxidase: 1400 U/l
   Polyoxyethylene-polyoxypropylene block polymer: 0.1% (w/v)
Second reagent composition
   BES buffer (pH 7.0): 100 mM
   Polyoxyethylene polycyclic phenyl ether: 1.0% (w/v)

### Analyte: LDL-C

First reagent composition
   PIPES buffer (pH 7.0): 50 mM
   Cholesterol esterase: 600 U/l
   Cholesterol oxidase: 500 U/l
   Polyoxyethylene polycyclic phenyl ether: 0.2% (w/v)
   BSA: 1.0% (w/v)
Second reagent composition
   PIPES buffer (pH 7.0): 50 mM
   Polyoxyethylene alkyl ether: 1.0% (w/v)

### Analyte: HDL3-C

First reagent composition
   BES buffer (pH 7.0): 100 mM
   Cholesterol esterase: 2000 U/l
   Cholesterol oxidase: 300 U/l
   Sphingomyelinase: 500 U/l
   Polyoxyethylene-polyoxypropylene block polymer: 0.05% (w/v)
   BSA: 1.0% (w/v)
Second reagent composition
   BES buffer (pH 7.0): 100 mM
   Polyoxyethylene polycyclic phenyl ether: 2.0% (w/v)

### Analyte: RLP-C

First reagent composition
   PIPES buffer (pH 6.8): 50 mM
   Cholesterol esterase (high molecular weight: CEBP-M (62 kDa)): 5000 U/l
   Cholesterol oxidase: 2500 U/l
   Sphingomyelinase: 2500 U/l
   Polyoxyethylene polycyclic phenyl ether: 0.1% (w/v)
   BSA: 1.0% (w/v)
Second reagent composition
   PIPES buffer (pH 6.8): 50 mM
   Cholesterol esterase (low molecular weight: CEN (29.5 kDa)): 4000 U/l
   Polyoxyethylene alkyl ether: 0.1% (w/v)

### Analyte: apoE-containing HDL-C

First reagent composition
   BES buffer (pH 7.0): 100 mM
   Cholesterol esterase: 800 U/l
   Cholesterol oxidase: 1600 U/l
   Polyoxyethylene polycyclic phenyl ether: 0.067% (w/v)
Second reagent composition
   BES buffer (pH 7.0): 100 mM
   Polyoxyethylene polycyclic phenyl ether: 1.0% (w/v)

### Comparative Example 1

The second reagent composition used in the second step was prepared with the addition of the ingredients described below to the reagent ingredients described above.

### Second reagent composition

Coupler (4-aminoantipyrine): 4.0 mM
Peroxidase: 2.4 U/ml
Potassium ferrocyanide: 0.11 mM
Sodium azide: 0.05% (w/v)

The second reagent composition was subjected to accelerated storage at 37°C for 2 weeks, spontaneous color development during storage was inspected by measuring the absorbance spectra at the wavelengths ranging from 320 nm to 480 nm.

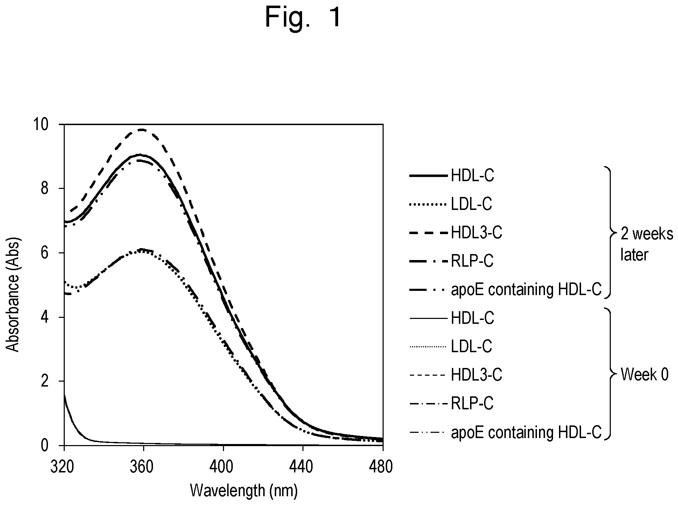

Fig. 1 shows the absorbance spectra immediately after preparation and after accelerated storage at 37°C for 2 weeks. As shown in Fig. 1, an increase in the absorbance was not observed in any analyte immediately after preparation (week 0). The reagent after accelerated storage at 37°C for 2 weeks exhibited a peak indicating the maximum absorbance at around 360 nm (the reagent became pale yellow). The results of accelerated storage at 37°C for 1 week were equivalent to the results of refrigeration for 1.5 years.

### Comparative Example 2

The first reagent composition used in the first step was prepared with the addition of the ingredients described below to the reagent ingredients described above.

### First reagent composition

Coupler (4-aminoantipyrine): 1.3 mM
Peroxidase: 1.7 U/ml
Potassium ferrocyanide: 0.04 mM

The first reagent composition was subjected to accelerated storage at 37°C for 2 weeks, spontaneous color development during storage was inspected by measuring the absorbance spectra at the wavelengths ranging from 320 nm to 480 nm.

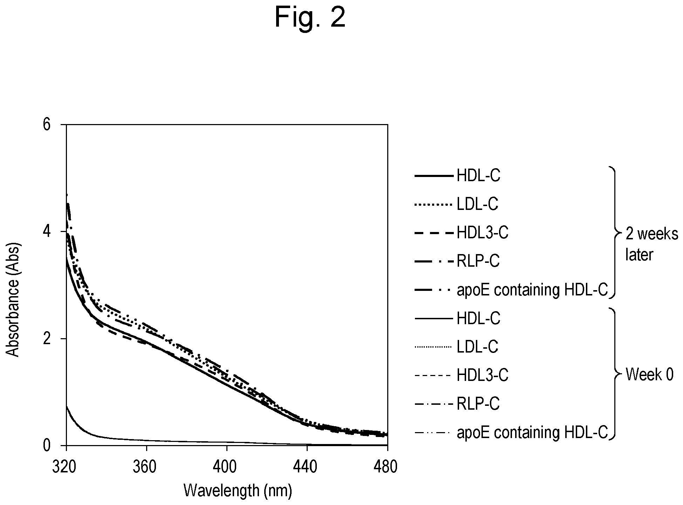

Fig. 2 shows the absorbance spectra immediately after preparation and after accelerated storage at 37°C for 2 weeks. As shown in Fig. 2, an increase in the absorbance was not observed in any analyte immediately after preparation (week 0). The reagent after accelerated storage at 37°C exhibited a peak indicating the maximum absorbance at around 360 nm (the reagent became pale yellow).

### Example 1

The first reagent composition used in the first step and the second reagent composition used in the second step were prepared with the addition of the ingredients described below to the reagent ingredients described above.

### First reagent composition

Catalase: 1200 U/ml
4-Aminoantipyrine: 1.3 mM

### Second reagent composition

Hydrogen donors
(HDAOS: HDL-C, apoE-containing HDL-C): 2.1 mM
(TOOS: LDL-C, HDL3-C, remnant-C): 6.0 mM
Potassium ferrocyanide: 0.11 mM
Peroxidase: 5.0 U/ml
Sodium azide: 0.05% (w/v)

The first reagent composition and the second reagent composition were subjected to accelerated storage at 37°C for 2 weeks, spontaneous color development during storage was inspected by measuring the absorbance spectra at the wavelengths ranging from 320 nm to 480 nm, and the results were compared to the results of Comparative Examples 1 and 2.

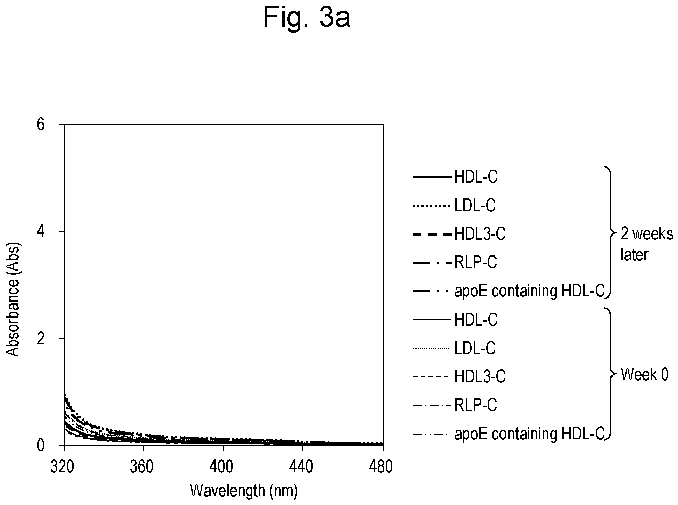

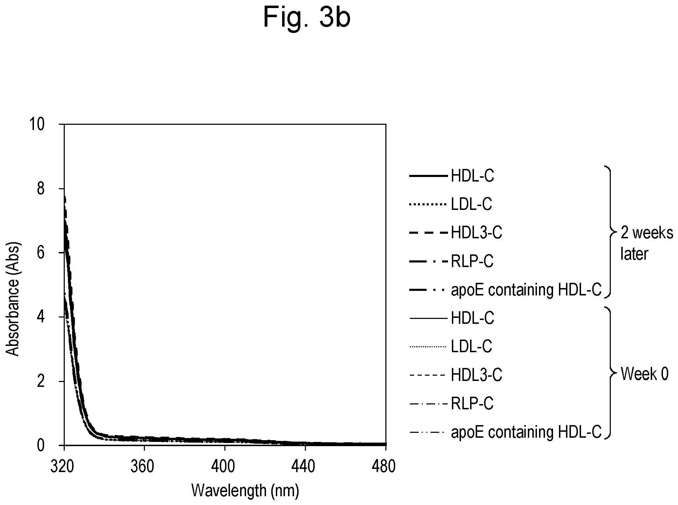

Fig. 3a shows the absorbance spectra of the first reagent composition and Fig. 3b shows the absorbance spectra of the second reagent composition immediately after preparation and after accelerated storage at 37°C for 2 weeks. As shown in Fig. 3a and Fig. 3b, no significant differences were observed between the results immediately after preparation (week 0) and the results after accelerated storage at 37°C for 2 weeks, and the peak indicating the maximum absorbance at around 360 nm was reduced compared to Comparative Examples 1 and 2 or disappeared.

### Example 2

The first reagent composition used in the first step and the second reagent composition used in the second step were prepared with the addition of the ingredients described below to the reagent ingredients described above.

### First reagent composition

Peroxidase: 1.7 U/ml
4-Aminoantipyrine: 1.3 mM

### Second reagent composition

Hydrogen donors
(HDAOS: HDL-C, apoE-containing HDL-C): 2.1 mM
(TOOS: LDL-C, HDL3-C, remnant-C): 6.0 mM
Potassium ferrocyanide: 0.11 mM

The first reagent composition and the second reagent composition were subjected to accelerated storage at 37°C for 2 weeks, spontaneous color development during storage was inspected by measuring the absorbance spectra at the wavelengths ranging from 320 nm to 480 nm, and the results were compared to the results of Comparative Examples 1 and 2.

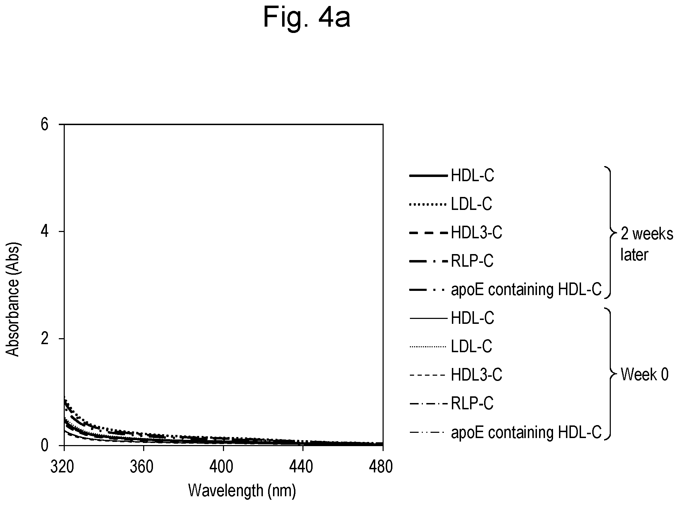

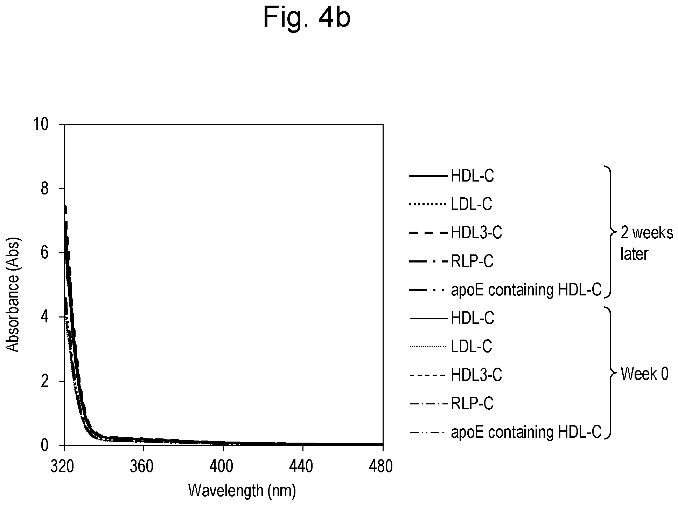

Fig. 4a shows the absorbance spectra of the first reagent composition and Fig. 4b shows the absorbance spectra of the second reagent composition immediately after preparation and after accelerated storage at 37°C for 2 weeks. As shown in Fig. 4a and Fig. 4b, no significant differences were observed between the results immediately after preparation (week 0) and the results after accelerated storage at 37°C for 2 weeks, and the peak indicating the maximum absorbance at around 360 nm was reduced compared to Comparative Examples 1 and 2 or disappeared.

### Example 3

The first reagent composition used in the first step and the second reagent composition used in the second step were prepared with the addition of the ingredients described below to the reagent ingredients described above.

### First reagent composition

4-Aminoantipyrine: 1.3 mM
Potassium ferrocyanide: 0.04 mM
Catalase: 1200 U/ml

### Second reagent composition

Hydrogen donors
(HDAOS: HDL-C, apoE-containing HDL-C): 2.1 mM
(TOOS: LDL-C, HDL3-C, remnant-C): 6.0 mM
Sodium azide: 0.05% (w/v)
Peroxidase: 5.0 U/ml

The first reagent composition and the second reagent composition were subjected to accelerated storage at 37°C for 2 weeks, spontaneous color development during storage was inspected by measuring the absorbance spectra at the wavelengths ranging from 320 nm to 480 nm, and the results were compared to the results of Comparative Examples 1 and 2.

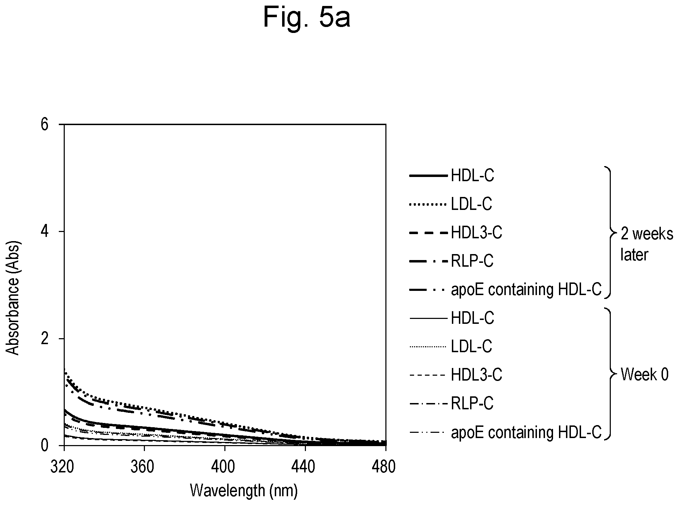

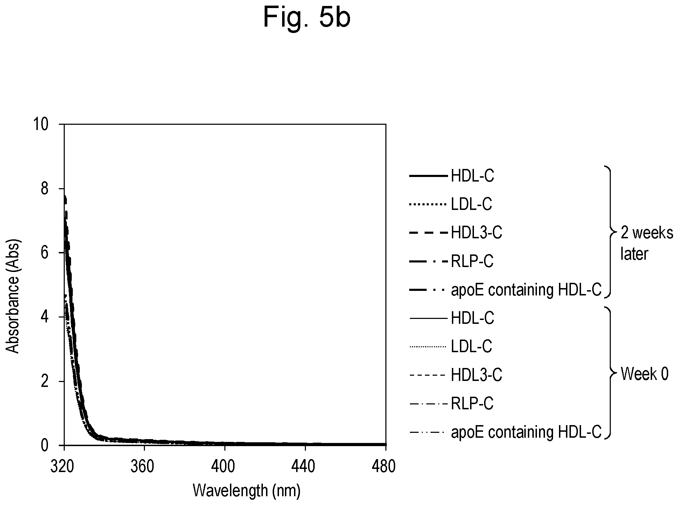

Fig. 5a shows the absorbance spectra of the first reagent composition and Fig. 5b shows the absorbance spectra of the second reagent composition immediately after preparation and after accelerated storage at 37°C for 2 weeks. As shown in Fig. 5a and Fig. 5b, no significant differences were observed between the results immediately after preparation (week 0) and the results after accelerated storage at 37°C for 2 weeks, and the peak indicating the maximum absorbance at around 360 nm was reduced compared to Comparative Examples 1 and 2 or disappeared.

### Example 4

The first reagent composition used in the first step and the second reagent composition used in the second step were prepared with the addition of the ingredients described below to the reagent ingredients described above.

### First reagent composition

Peroxidase: 1.7 U/ml
Hydrogen donors
(HDAOS: HDL-C, apoE-containing HDL-C): 0.7 mM
(TOOS: LDL-C, HDL3-C, remnant-C): 2.0 mM

### Second reagent composition

4-Aminoantipyrine: 4.0 mM
Potassium ferrocyanide: 0.11 mM

The first reagent composition and the second reagent composition were subjected to accelerated storage at 37°C for 2 weeks, spontaneous color development during storage was inspected by measuring the absorbance spectra at the wavelengths ranging from 320 nm to 480 nm, and the results were compared to the results of Comparative Examples 1 and 2.

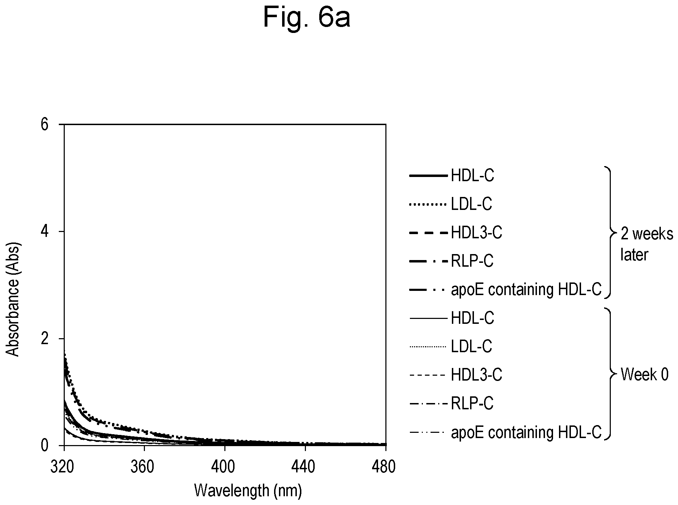

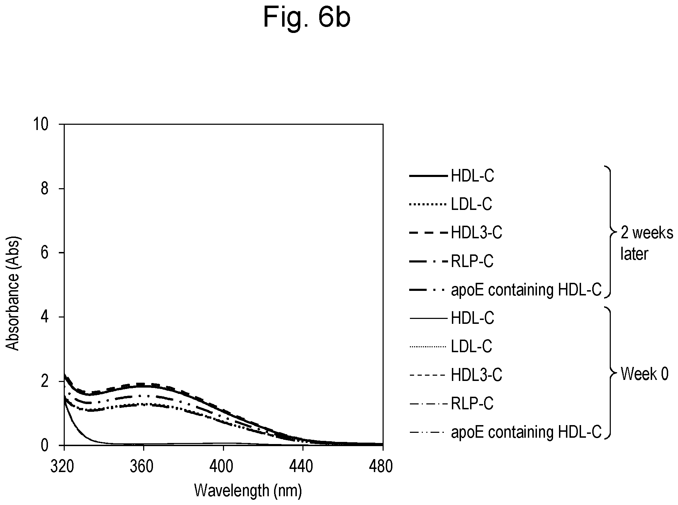

Fig. 6a shows the absorbance spectra of the first reagent composition and Fig. 6b shows the absorbance spectra of the second reagent composition immediately after preparation and after accelerated storage at 37°C for 2 weeks. Concerning the first reagent composition (Fig. 6a), no significant differences were observed between the results immediately after preparation (week 0) and the results after accelerated storage at 37°C for 2 weeks. Concerning the second reagent composition (Fig. 6b), the peak indicating the maximum absorbance at around 360 nm was somewhat higher 2 weeks after storage, and the peak indicating the maximum absorbance at around 360 nm was reduced compared to Comparative Examples 1 and 2 or disappeared.

### Example 5

The first reagent composition used in the first step and the second reagent composition used in the second step were prepared with the addition of the ingredients described below to the reagent ingredients described above.

### First reagent composition

Hydrogen donors
(HDAOS: HDL-C, apoE-containing HDL-C): 0.7 mM
(TOOS: LDL-C, HDL3-C, remnant-C): 2.0 mM
Potassium ferrocyanide: 0.04 mM
Catalase: 1200 U/ml

### Second reagent composition

4-Aminoantipyrine: 4.0 mM
Peroxidase: 5.0 U/ml
Sodium azide: 0.05% (w/v)

The first reagent composition and the second reagent composition were subjected to accelerated storage at 37°C for 2 weeks, spontaneous color development during storage was inspected by measuring the absorbance spectra at the wavelengths ranging from 320 nm to 480 nm, and the results were compared to the results of Comparative Examples 1 and 2.

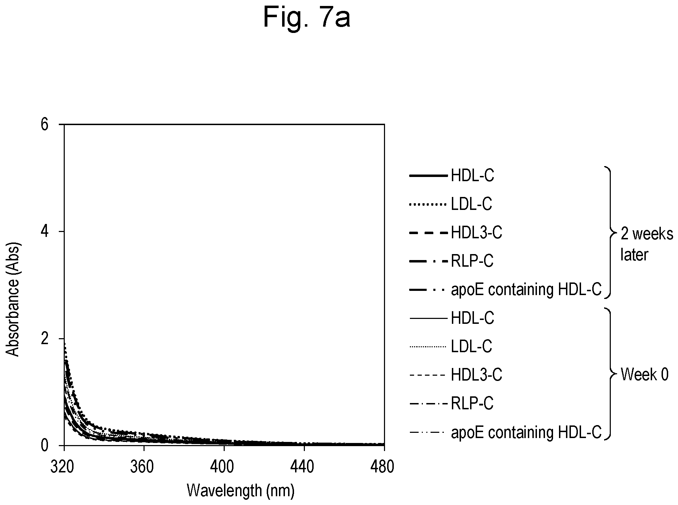

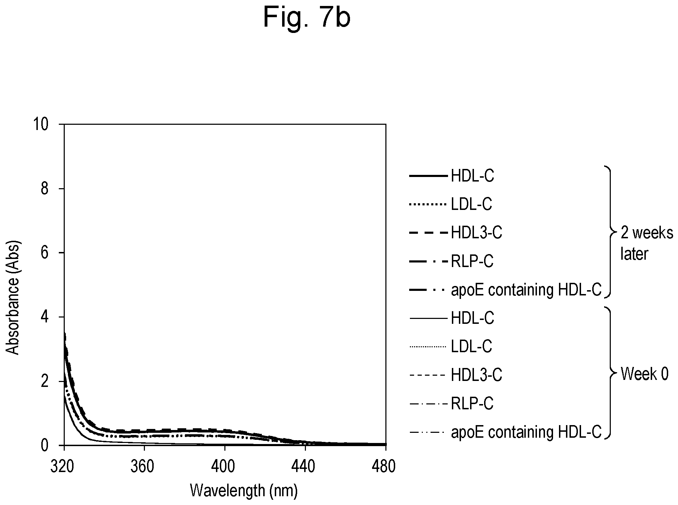

Fig. 7a shows the absorbance spectra of the first reagent composition and Fig. 7b shows the absorbance spectra of the second reagent composition immediately after preparation and after accelerated storage at 37°C for 2 weeks. As shown in Fig. 7a and Fig. 7b, no significant differences were observed between the results immediately after preparation (week 0) and the results after accelerated storage at 37°C for 2 weeks, and the peak indicating the maximum absorbance at around 360 nm was reduced compared to Comparative Examples 1 and 2 or disappeared.

### Example 6

The first reagent composition used in the first step and the second reagent composition used in the second step were prepared with the addition of the ingredients described below to the reagent ingredients described above.

### First reagent composition

Hydrogen donors
(HDAOS: HDL-C, apoE-containing HDL-C): 0.7 mM
(TOOS: LDL-C, HDL3-C, remnant-C): 2.0 mM
Potassium ferrocyanide: 0.04 mM
Peroxidase: 1.7 U/ml

### Second reagent composition

4-Aminoantipyrine: 4.0 mmol/l

The first reagent composition and the second reagent composition were subjected to accelerated storage at 37°C for 2 weeks, spontaneous color development during storage was inspected by measuring the absorbance spectra at the wavelengths ranging from 320 nm to 480 nm, and the results were compared to the results of Comparative Examples 1 and 2.

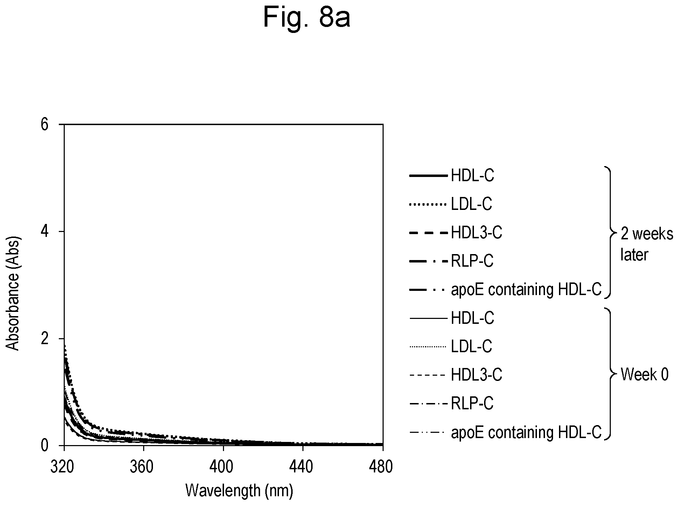

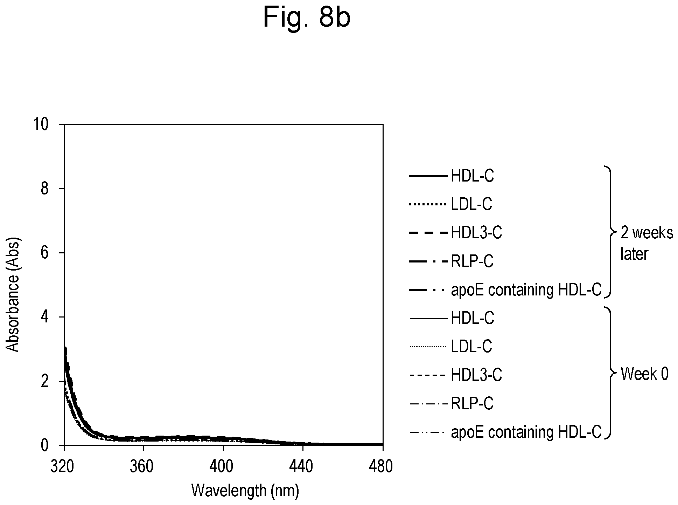

Fig. 8a shows the absorbance spectra of the first reagent composition and Fig. 8b shows the absorbance spectra of the second reagent composition immediately after preparation and after accelerated storage at 37°C for 2 weeks. As shown in Fig. 8a and Fig. 8b, no significant differences were observed between the results immediately after preparation (week 0) and the results after accelerated storage at 37°C for 2 weeks, and the peak indicating the maximum absorbance at around 360 nm was reduced compared to Comparative Examples 1 and 2 or disappeared.

### Example 7

The first reagent composition used in the first step and the second reagent composition used in the second step were prepared with the addition of the ingredients described below to the reagent ingredients described above.

### First reagent composition

4-Aminoantipyrine: 1.3 mM
Peroxidase: 1.7 U/ml
Catalase: 1200 U/ml

### Second reagent composition

Hydrogen donors
(HDAOS: HDL-C, apoE-containing HDL-C): 2.1 mM
(TOOS: LDL-C, HDL3-C, remnant-C): 6.0 mM
Potassium ferrocyanide: 0.11 mM
Sodium azide: 0.05% (w/v)

The first reagent composition and the second reagent composition were subjected to accelerated storage at 37°C for 2 weeks, spontaneous color development during storage was inspected by measuring the absorbance spectra at the wavelengths ranging from 320 nm to 480 nm, and the results were compared to the results of Comparative Examples 1 and 2.

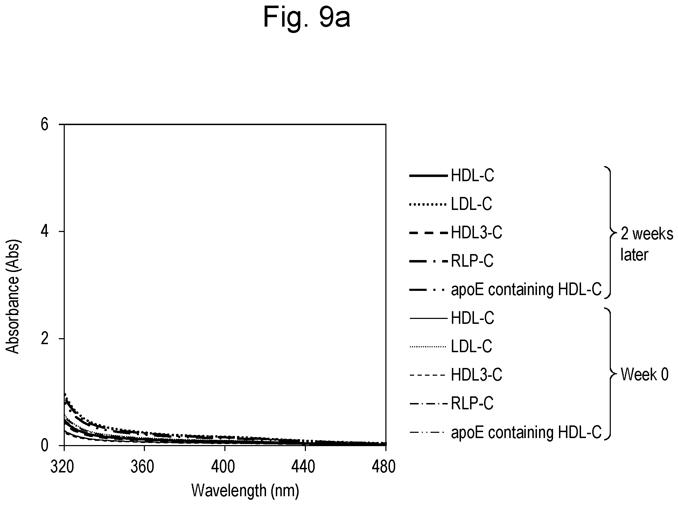

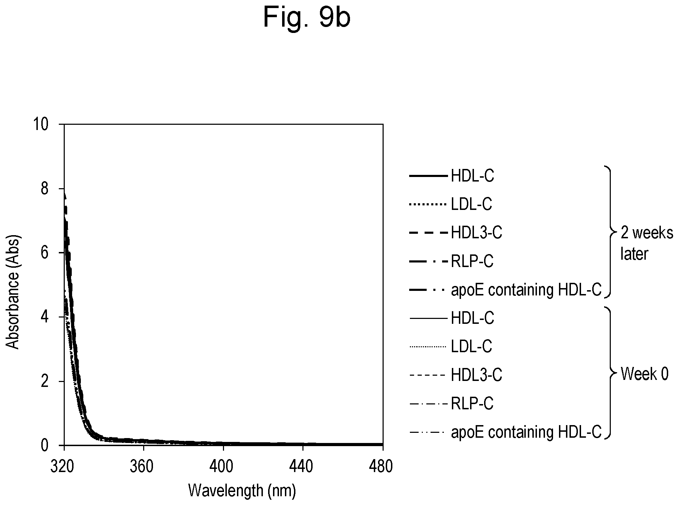

Fig. 9a shows the absorbance spectra of the first reagent composition and Fig. 9b shows the absorbance spectra of the second reagent composition immediately after preparation and after accelerated storage at 37°C for 2 weeks. As shown in Fig. 9a and Fig. 9b, no significant differences were observed between the results immediately after preparation (week 0) and the results after accelerated storage at 37°C for 2 weeks, and the peak indicating the maximum absorbance at around 360 nm was reduced compared to Comparative Examples 1 and 2 or disappeared.

### Example 8

The first reagent composition used in the first step and the second reagent composition used in the second step were prepared with the addition of the ingredients described below to the reagent ingredients described above.

### First reagent composition

Hydrogen donors
(HDAOS: HDL-C, apoE-containing HDL-C): 0.7 mM
(TOOS: LDL-C, HDL3-C, remnant-C): 2.0 mM
Peroxidase: 1.7 U/ml
Catalase: 1200 U/ml

### Second reagent composition

4-Aminoantipyrine: 4.0 mM
Potassium ferrocyanide: 0.11 mM
Sodium azide: 0.05% (w/v)

The first reagent composition and the second reagent composition were subjected to accelerated storage at 37°C for 2 weeks, spontaneous color development during storage was inspected by measuring the absorbance spectra at the wavelengths ranging from 320 nm to 480 nm, and the results were compared to the results of Comparative Examples 1 and 2.

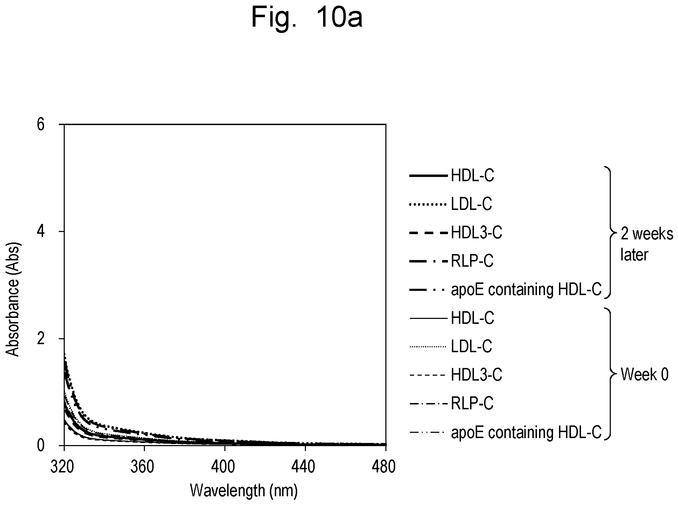

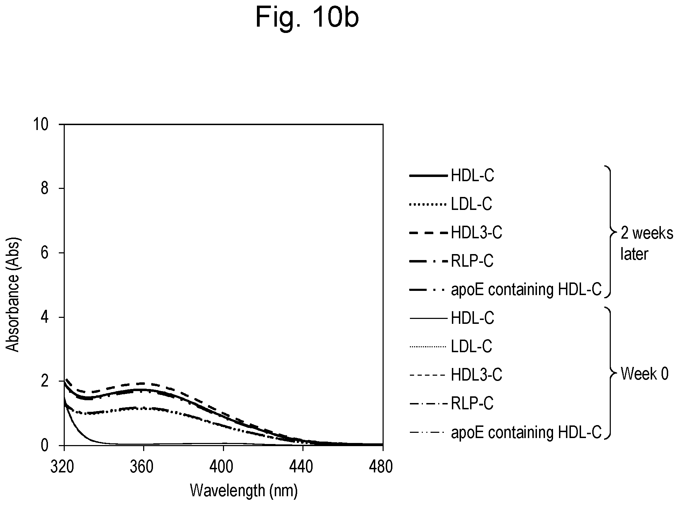

Fig. 10a shows the absorbance spectra of the first reagent composition and Fig. 10b shows the absorbance spectra of the second reagent composition immediately after preparation and after accelerated storage at 37°C for 2 weeks. Concerning the first reagent composition (Fig. 10a), no significant differences were observed between the results immediately after preparation (week 0) and the results after accelerated storage at 37°C for 2 weeks. Concerning the second reagent composition (Fig. 10b), the peak indicating the maximum absorbance at around 360 nm was somewhat higher 2 weeks after storage, and the peak indicating the maximum absorbance at around 360 nm was reduced compared to Comparative Examples 1 and 2 or disappeared.

### Example 9

The first reagent composition used in the first step and the second reagent composition used in the second step were prepared with the addition of the ingredients described below to the reagent ingredients described above.

### First reagent composition

Hydrogen donors
(HDAOS: HDL-C, apoE-containing HDL-C): 0.7 mM
(TOOS: LDL-C, HDL3-C, remnant-C): 2.0 mM
Potassium ferrocyanide: 0.04 mM
Peroxidase: 1.7 U/ml
Catalase: 1200 U/ml

### Second reagent composition

4-Aminoantipyrine: 4.0 mM
Sodium azide: 0.05% (w/v)

The first reagent composition and the second reagent composition were subjected to accelerated storage at 37°C for 1 week or 2 weeks, spontaneous color development during storage was inspected by measuring the absorbance spectra at the wavelengths ranging from 320 nm to 480 nm, and the results were compared to the results of Comparative Examples 1 and 2.

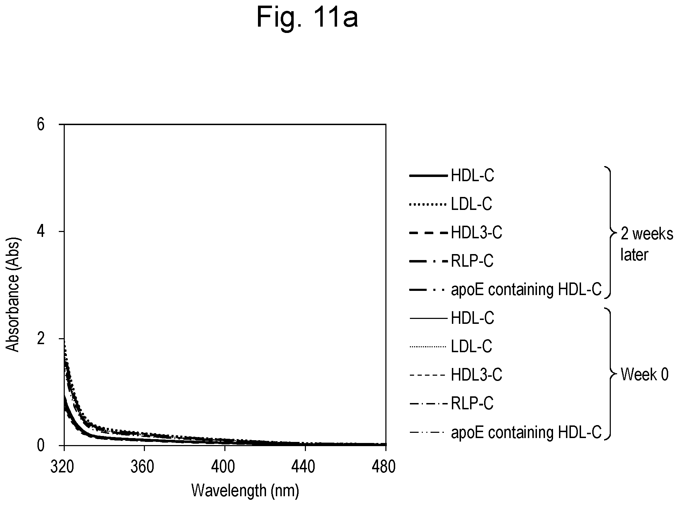

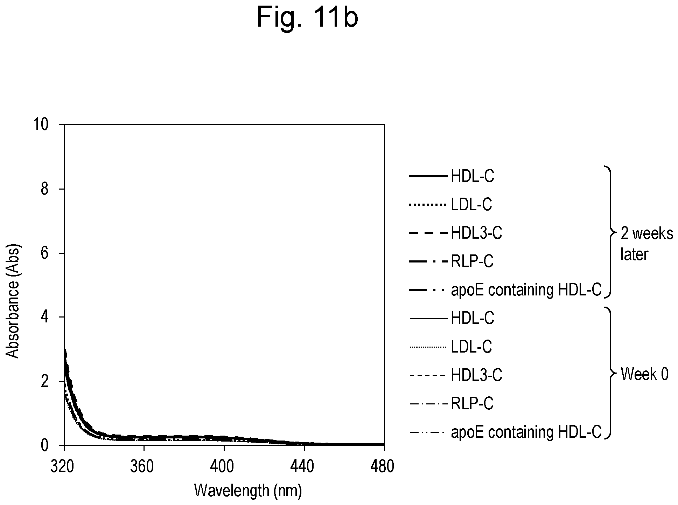

Fig. 11a shows the absorbance spectra of the first reagent composition and Fig. 11b shows the absorbance spectra of the second reagent composition immediately after preparation and after accelerated storage at 37°C for 2 weeks. As shown in Fig. 11a and Fig. 11b, no significant differences were observed between the results immediately after preparation (week 0) and the results after accelerated storage at 37°C for 2 weeks, and the peak indicating the maximum absorbance at around 360 nm was reduced compared to Comparative Examples 1 and 2 or disappeared.

### Examination 1

With the use of the kits for quantification of Example 1 to Example 9, lipoprotein cholesterol was assayed. For comparison, HDL-C, LDL-C, and HDL3-C were assayed using the reagents for measuring the lipoprotein cholesterol level, HDL-EX "Seiken," LDL-EX "Seiken," and HDL3-C "SEIKEN" (Denka Seiken Co., Ltd.), respectively, apoE-containing HDL-C was assayed in accordance with the method described in Ann. Clin. Biochem., 56, 123-132, 2019, remnant-C was assayed in accordance with the method described in Ann. Clin. Biochem., 56, 123-132, 2019, and the lipoprotein cholesterol levels were then compared. Table 1 shows the correlation coefficients between the results of measurement of lipoprotein cholesterol obtained in the examples and the results of measurement obtained in the comparative examples concerning the same sample.

As shown in Table 1, a satisfactory correlation was observed between the method of the examples according to the present invention and the comparative method.

**Table 1**

| Example | HDL-C | LDL-C | HDL3-C | RLP-C | apoE-containing HDL-C |
|---|---|---|---|---|---|
| Example 1 | 0.999 | 0.998 | 0.995 | 0.994 | 0.996 |
| Example 2 | 0.991 | 0.992 | 0.989 | 0.988 | 0.989 |
| Example 3 | 0.993 | 0.994 | 0.992 | 0.991 | 0.994 |
| Example 4 | 0.989 | 0.988 | 0.984 | 0.982 | 0.988 |
| Example 5 | 0.998 | 0.998 | 0.996 | 0.994 | 0.996 |
| Example 6 | 0.984 | 0.986 | 0.986 | 0.982 | 0.987 |
| Example 7 | 0.992 | 0.990 | 0.991 | 0.989 | 0.991 |
| Example 8 | 0.994 | 0.992 | 0.991 | 0.990 | 0.994 |
| Example 9 | 0.990 | 0.990 | 0.991 | 0.989 | 0.991 |

The results of the examination described above demonstrate that the kit for quantification of the present invention enables satisfactory detection of HDL-C, LDL-C, HDL3-C, remnant-C, and apoE-containing HDL-C.

### Industrial Applicability

With the use of the kit and the method of the present invention, lipoprotein cholesterol can be quantified without damaging reagent stability.

All publications, patents, and patent applications cited herein are incorporated herein by reference in their entirety.

## Claims

1. A kit for quantification of lipoprotein cholesterol in a sample obtained from a subject used in a method for quantification of lipoprotein cholesterol in two steps comprising:
(1) a first reagent composition having cholesterol esterase and cholesterol oxidase and leading lipoprotein cholesterol other than the analyte to the outside of the reaction system; and
(2) a second reagent composition for quantifying the analyte lipoprotein cholesterol,
wherein either the first reagent composition or the second reagent composition comprises at least a coupler, an iron complex, peroxidase, a hydrogen donor, and a surfactant, provided that the coupler and the hydrogen donor are not allowed to be present in the same reagent composition, and the coupler, the iron complex, and peroxidase are not allowed to be present together in either of the first reagent composition or the second reagent composition.

2. A kit for quantification of lipoprotein cholesterol in a sample obtained from a subject used in the method for quantification of lipoprotein cholesterol in two steps comprising:
(1) a first reagent composition having cholesterol esterase and cholesterol oxidase and leading lipoprotein cholesterol other than the analyte to the outside of the reaction system; and
(2) a second reagent composition for quantifying lipoprotein cholesterol,
wherein either the first reagent composition or the second reagent composition comprises at least a coupler, an iron complex, peroxidase, a hydrogen donor, and a surfactant, provided that the coupler and the hydrogen donor are not allowed to be present in the same reagent composition, and the coupler and the iron complex are not allowed to be present together in either of the first reagent composition or the second reagent composition.

3. The kit for quantifying lipoprotein cholesterol according to Claim 1 or 2, wherein the first reagent composition comprises catalase and a coupler and the second reagent composition comprises a hydrogen donor, an iron complex, and peroxidase.

4. The kit for quantifying lipoprotein cholesterol according to Claim 1 or 2, wherein the first reagent composition comprises peroxidase and a coupler and the second reagent composition comprises a hydrogen donor and an iron complex.

5. The kit for quantifying lipoprotein cholesterol according to Claim 1, wherein the first reagent composition comprises catalase, a coupler, and an iron complex and the second reagent composition comprises a hydrogen donor and peroxidase.

6. The kit for quantifying lipoprotein cholesterol according to Claim 1, wherein the first reagent composition comprises peroxidase and a hydrogen donor and the second reagent composition comprises a coupler and an iron complex.

7. The kit for quantifying lipoprotein cholesterol according to Claim 1 or 2, wherein the first reagent composition comprises catalase, a hydrogen donor, and an iron complex and the second reagent composition comprises peroxidase and a coupler.

8. The kit for quantifying lipoprotein cholesterol according to Claim 1 or 2, wherein the first reagent composition comprises peroxidase, a hydrogen donor, and an iron complex and the second reagent composition comprises a coupler.

9. The kit for quantifying lipoprotein cholesterol according to any one of Claims 4, 6, and 8, wherein the first reagent composition further comprises catalase.

10. The kit for quantifying lipoprotein cholesterol according to any one of Claims 1 to 9, wherein the first reagent composition further comprises a surfactant that acts on lipoproteins other than the analyte and the second reagent composition further comprises a surfactant that acts at least on the analyte lipoproteins.

11. The kit for quantifying lipoprotein cholesterol according to any one of Claims 1 to 10, wherein the analyte lipoprotein cholesterol is LDL cholesterol, HDL cholesterol, HDL3 cholesterol, remnant cholesterol, or apoE-containing HDL cholesterol.

12. The kit for quantifying lipoprotein cholesterol according to Claim 10 or 11, wherein the surfactant contained in the first reagent composition comprises a polyoxyethylene polycyclic phenyl ether derivative or a polyoxyethylene-polyoxypropylene block polymer.

13. The kit for quantifying lipoprotein cholesterol according to Claim 12, wherein the polyoxyethylene polycyclic phenyl ether derivative includes a polyoxyethylene benzyl phenyl ether derivative and/or a polyoxyethylene styrenated phenyl ether derivative.

14. A method for quantification of lipoprotein cholesterol in a sample obtained from a subject in two steps comprising:
(1) a first step of leading lipoprotein cholesterol other than the analyte to the outside of the reaction system in the presence of cholesterol esterase and cholesterol oxidase; and
(2) a second step of quantifying the analyte lipoprotein cholesterol remaining after the first step,
wherein, in either the step (1) or (2), at least the coupler, the iron complex, the peroxidase, the hydrogen donor, and the surfactant are used, and the coupler and the hydrogen donor are not used in the same step, and
in either the step (1) or (2), the coupler, the iron complex, and peroxidase are not used simultaneously.

15. A method for quantification of lipoprotein cholesterol in a sample obtained from a subject in two steps comprising:
(1) a first step of leading lipoprotein cholesterol other than the analyte to the outside of the reaction system in the presence of cholesterol esterase and cholesterol oxidase; and
(2) a second step of quantifying the analyte lipoprotein cholesterol remaining after the first step,
wherein, in either the step (1) or (2), the coupler and the iron complex are not used simultaneously.

16. The method for quantification of lipoprotein cholesterol according to Claim 14 or 15, wherein the first step involves the use of the catalase and the coupler and the second step involves the use of the hydrogen donor, the iron complex, and the peroxidase.

17. The method for quantification of lipoprotein cholesterol according to Claim 14 or 15, wherein the first step involves the use of the peroxidase and the coupler and the second step involves the use of the hydrogen donor and the iron complex.

18. The method for quantification of lipoprotein cholesterol according to Claim 14, wherein the first step involves the use of the catalase, the coupler, and the iron complex and the second step involves the use of the hydrogen donor and the peroxidase.

19. The method for quantification of lipoprotein cholesterol according to Claim 14, wherein the first step involves the use of the peroxidase and the hydrogen donor and the second step involves the use of the coupler and the iron complex.

20. The method for quantification of lipoprotein cholesterol according to Claim 14 or 15, wherein the first step involves the use of the catalase, the hydrogen donor, and the iron complex and the second step involves the use of the peroxidase and the coupler.

21. The method for quantification of lipoprotein cholesterol according to Claim 14 or 15, wherein the first step involves the use of the peroxidase, the hydrogen donor, and the iron complex and the second step involves the use of the coupler.

22. The method for quantification of lipoprotein cholesterol according to any one of Claims 17, 19, and 21, wherein the first step further involves the use of the catalase.

23. The method for quantification of lipoprotein cholesterol according to any one of Claims 14 to 22, wherein the first step further involves the use of a surfactant that acts on lipoproteins other than the analyte and the second step further involves the use of a surfactant that acts at least on the analyte lipoproteins.

24. The method for quantification of lipoprotein cholesterol according to any one of Claims 14 to 23, wherein the analyte lipoprotein cholesterol is LDL cholesterol, HDL cholesterol, HDL3 cholesterol, remnant cholesterol, or apoE-containing HDL cholesterol.
